# EUROPEAN PATENT SPECIFICATION

(11) **EP 4 397 230 B1**
(45) Date of publication and mention of the grant of the patent: **09.12.2026**
(21) Application number: 24177131.0
(22) Date of filing: 15.05.2020
(51) Int. Cl.: A61B 5/00

(54) **SEIZURE DETECTION DEVICE**
VORRICHTUNG ZUR ERKENNUNG VON ANFÄLLEN
DISPOSITIF DE DÉTECTION DE CRISE

(30) Priority: 15.05.2019 US 201962848319 P; 14.05.2020 US 202016874229
(43) Date of publication of application: 10.07.2024
(62) Document number(s) of the earlier application(s) in accordance with Art. 76 EPC: 20805666.3 / 3 968 848
(73) Proprietor: Know Biological, Inc., Milton, Georgia 30004 (US); National Technology & Engineering Solutions of Sandia, LLC, Albuquerque, NM 87123 (US)
(72) Inventor: ARNOLD, Gary Stephen, Cumming, 30040 (US); MOORMAN, Matthew Wallace, Albuquerque, 87106 (US); WHITING, Joshua Jonathan, Albuquerque, 87185-0114 (US)
(74) Representative: Page White Farrer

(56) References cited:
- WO-A1-2018/173060
- WO-A1-2019/008973
- WO-A1-2021/050678
- US-A1- 2020 008 756
- DAVIS PHILIP R.N.: "The Investigation of Human Scent from Epileptic Patients for the Identification of a Biomarker for Epileptic Seizures", FIU ELECTRONIC THESES AND DISSERTATIONS. 3520, 31 December 2017 (2017-12-31), XP093040545, Retrieved from the Internet <URL:https://digitalcommons.fiu.edu/etd/3520> [retrieved on 20230420], DOI: 10.25148/etd.FIDC004043
- JIANG RUIFEN ET AL: "A non-invasive method forin vivoskin volatile compounds sampling", ANALYTICA CHIMICA ACTA, ELSEVIER, AMSTERDAM, NL, vol. 804, 17 October 2013 (2013-10-17), pages 111 - 119, XP028780792, ISSN: 0003-2670, DOI: 10.1016/J.ACA.2013.09.056
- VERONIKA RUZSANYI ET AL: "Ion mobility spectrometry for detection of skin volatiles", JOURNAL OF CHROMATOGRAPHY B, vol. 911, 1 December 2012 (2012-12-01), NL, pages 84 - 92, XP055564046, ISSN: 1570-0232, DOI: 10.1016/j.jchromb.2012.10.028
- PERYCZ URSZULA ET AL: "Coupling Gas Chromatography with Ion Mobility Spectrometry", LC GC EUROPE, vol. 29, no. 6, 20 May 2016 (2016-05-20), pages 294 - 303, XP055869951
- GAVIN JAMES BLACKBURN: "Breath analysis: methodology towards a fieldable breath analysis device", THESIS LOUGHBOROUGH UNIVERSITY, 1 January 2011 (2011-01-01), XP055636778, Retrieved from the Internet <URL:https://repository.lboro.ac.uk/articles/thesis/Breath_analysis_methodology_towards_a_fieldable_breath_analysis_device/9396482?file=17011265> [retrieved on 20191029]

## Description

### STATEMENT OF GOVERNMENT INTEREST

This invention was made with government support under Contract No. DE-NA0003525 awarded by the United States Department of Energy/National Nuclear Security Administration. The government has certain rights in the invention.

### TECHNICAL FIELD

This disclosure relates to medical devices. More specifically, this disclosure relates to a seizure detection device.

### BACKGROUND

Epilepsy is the most common neurological disorder in the world after migraine, stroke, and Alzheimer's disease. It is a disorder of the central nervous system, not caused by an underlying, treatable medical condition, characterized by recurring periods of altered brain function caused by abnormal or excessive electrical discharges in the brain, resulting in what is commonly called a seizure. It is one of the world's oldest recognized health conditions, with recorded occurrences dating back to 4,000 BC.

Worldwide, there are nearly 65 million people who have epilepsy, with more than 3.5 million in the U.S. alone. Worldwide, there are 2.4 million new cases of epilepsy each year, with more than 150,000 new cases each year in the U.S. alone. Over a lifetime, more than one in twenty-six people with be diagnosed with the disease. Medication and medical intervention can control seizures in approximately two-thirds of patients, with the remaining one-third experiencing uncontrolled and unpredictable seizure episodes. There are estimated to be nearly one million deaths directly related to epilepsy each year worldwide, including some 50,000 deaths in the U.S each year.

Each year, approximately 80 people out of every 100,000 in the general population will experience new-onset seizures, and approximately 60% of these will have repeated episodes leading to the diagnosis of epilepsy. Misunderstanding, prejudice, and social humiliation have always surrounded epilepsy. This continues in most countries today and can significantly impact the quality of life for people with epilepsy.

The social consequences of epilepsy are often more impactful than the seizures themselves. The lack of predictability inherent in epilepsy is devastating. Never knowing when a seizure might strike imposes major limitations in family, social, educational, and vocational activities. In addition to the potential of serious injury from falls and other accidents during seizures, the societal stigma attached to epilepsy and its unpredictability that can cause significant demoralization, irritation, and anxiety. Frustratingly, studies have shown that increased anxiety can lead to increased incidence of seizures, and increased seizures can lead to an even greater increase in chronic anxiety.

In summation, unexpected seizures can result in accident, injury, embarrassment, and costly trips to the emergency room. They can be difficult to predict and can be dangerous, particularly in instances where the patient is unable to contact family, a friend, or medical personnel when needed. Furthermore, patients often must take daily prophylactic medications that can be toxic and can be accompanied by unpleasant, occasionally life-threatening side effects.

WO2019008973A1 discloses acquiring biological gas information showing the 2-ethyl-1-hexanol concentration released from the skin surface of a user together with time information. Information representing the upper limit of a normal range of 2-ethyl-1-hexanol per unit time period is read from a memory and time periods during which the 2-ethyl-1-hexanol concentration of the user exceeds the upper limit of the normal range are determined.GB2533093A discloses a solid-phase micro extraction (SPME) device which may be used in combination with a breath sampler. The device may be used as a test for a Helicobacter Pylori infection.

WO2018173060A1 describes methods of diagnosing and/or monitoring tuberculosis (TB) in a subject by analyzing a test sample comprising at least one volatile organic compound (VOC) or semi-volatile organic compound (SVOC) emitted or excreted from the skin of the subject. The test sample can be analyzed by a sensing unit comprising nanomaterials- and/or polymer-based sensors.

Davis Philip R.N., "The Investigation of Human Scent from Epileptic Patients for the Identification of a Biomarker for Epileptic Seizures", FIU Electronic Theses and Dissertations. 3520, doi:10.25148/etd.FIDC004043, (20171231), URL: https://digitalcommons.fiu.edu/etd/3520, discloses the use of headspace solid phase microextraction (HS-SPME) with gas chromatography-mass spectrometry (GC-MS) to analyze hand odor, saliva and breath samples from epileptic with and without seizure activity to determine if the human scent profiles resulting from a seizure event differs from the scent profiles in the absence of seizure activity.

Ruifen Jiang et al, "A non-invasive method for in vivo skin volatile compounds sampling" ANALYTICA CHIMICA ACTA, Vol. 804, 17 October 2013, Pages 111-119 discloses a non-invasive headspace sampling method for volatile compounds emanating from human skin, using thin film as the extraction phase format. The technique was applied to identify the biomarkers of garlic intake and alcohol ingestion.

Veronika Ruzsanyi, et al. "Ion mobility spectrometry for detection of skin volatiles" Journal of Chromatography, vol. 911, December 2012, Pages 84 to 92 discloses the investigation of volatile organic compounds (VOCs) released by humans through their skin in near real time using ion mobility spectrometry after gas chromatographic separation with a short multi-capillary column.

### SUMMARY

It is to be understood that this summary is not an extensive overview of the disclosure. This summary is exemplary and not restrictive, and it is intended neither to identify key or critical elements of the disclosure nor delineate the scope thereof. The sole purpose of this summary is to explain and exemplify certain concepts of the disclosure as an introduction to the following complete and extensive detailed description.

Described is a collector for a seizure detection device comprising a collector material configured to collect volatile organic compounds given off from a patient's skin; a wrapping configured to isolate the collector material from an external environment; a heater comprising a heating element and one or more flow channels between the heating element and the collector material, the heating element configured to emit a thermal pulse to desorb the volatile organic compounds from the collector material, wherein the heater is one of integrated with the wrapping and received between the wrapping and the collector material, wherein the heater is one of integrated with the wrapping and received between the wrapping and the collector material, wherein the heater is one of integrated with the wrapping and received between the wrapping and the collector material, wherein the heater is one of integrated with the wrapping and received between the wrapping and the collector material; and a mesh layer configured to prevent the collector material from contacting the patient's skin, wherein the collector material is received between the wrapping and the mesh layer.

The present invention is a seizure detection device as defined in claim 1.

Also described but not forming part of the claimed invention is a method of detecting a seizure comprising collecting volatile organic compounds with a collector material of a collector; separating each of the volatile organic compounds into its constituent chemicals with a gas chromatography column; ionizing the constituent chemicals to create ionized chemicals and detecting the ionized chemicals; and analyzing the ionized chemicals to identify seizure-indicative volatile organic compounds

A seizure detection device is also described, the seizure detection device comprising a collector configured to collect volatile organic compounds given off from a patient's skin wherein the collector comprises a chemically-clean wrapping configured to isolate the collector material from external environmental contaminants; a separator configured to separate mixtures of the volatile organic compounds into their constituent chemicals; and an identifier configured to ionize the constituent chemicals to create ionized chemicals and to process information about the ionized chemicals to identify specific volatile organic compounds indicative of a seizure; a heater comprising a heating element and one or more flow channels between the heating element and the collector material, the heating element configured to emit a thermal pulse to desorb the volatile organic compounds from the collector material, wherein the heater is one of integrated with the wrapping and received between the wrapping and the collector material, wherein the heater is one of integrated with the wrapping and received between the wrapping and the collector material, wherein the heater is one of integrated with the wrapping and received between the wrapping and the collector material, wherein the heater is one of integrated with the wrapping and received between the wrapping and the collector material.

Various implementations described in the present disclosure may include additional systems, methods, features, and advantages, which may not necessarily be expressly disclosed herein but will be apparent to one of ordinary skill in the art upon examination of the following detailed description and accompanying drawings.

### BRIEF DESCRIPTION OF THE DRAWINGS

The features and components of the following figures are illustrated to emphasize the general principles of the present disclosure. Corresponding features and components throughout the figures may be designated by matching reference characters for the sake of consistency and clarity.
FIG. 1 is a top perspective view of a seizure detection device, in accordance with one aspect of the present disclosure.
FIG. 2 is a cross-sectional view of a collector of the seizure detection device of FIG. 1, taken along line 2-2 of FIG.1.

### DETAILED DESCRIPTION

The present disclosure can be understood more readily by reference to the following detailed description, examples, drawings, and claims, and the previous and following description. However, before the present devices, systems, and/or methods are disclosed and described, it is to be understood that this disclosure is not limited to the specific devices, systems, and/or methods disclosed unless otherwise specified, and, as such, can, of course, vary. It is also to be understood that the terminology used herein is for the purpose of describing particular aspects only and is not intended to be limiting.

The following description is provided as an enabling teaching of the present devices, systems, and/or methods in its best, currently known aspect. To this end, those skilled in the relevant art will recognize and appreciate that many changes can be made to the various aspects of the present devices, systems, and/or methods described herein, while still obtaining the beneficial results of the present disclosure. It will also be apparent that some of the desired benefits of the present disclosure can be obtained by selecting some of the features of the present disclosure without utilizing other features. Accordingly, those who work in the art will recognize that many modifications and adaptations to the present disclosure are possible and can even be desirable in certain circumstances and are a part of the present disclosure. Thus, the following description is provided as illustrative of the principles of the present disclosure and not in limitation thereof.

As used throughout, the singular forms "a," "an" and "the" include plural referents unless the context clearly dictates otherwise. Thus, for example, reference to "an element" can include two or more such elements unless the context indicates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

For purposes of the current disclosure, a material property or dimension measuring about X or substantially X on a particular measurement scale measures within a range between X plus an industry-standard upper tolerance for the specified measurement and X minus an industry-standard lower tolerance for the specified measurement. Because tolerances can vary between different materials, processes and between different models, the tolerance for a particular measurement of a particular component can fall within a range of tolerances.

As used herein, the terms "optional" or "optionally" mean that the subsequently described event or circumstance can or cannot occur, and that the description includes instances where said event or circumstance occurs and instances where it does not.

The word "or" as used herein means any one member of a particular list and also includes any combination of members of that list. Further, one should note that conditional language, such as, among others, "can," "could," "might," or "may," unless specifically stated otherwise, or otherwise understood within the context as used, is generally intended to convey that certain aspects include, while other aspects do not include, certain features, elements and/or steps. Thus, such conditional language is not generally intended to imply that features, elements and/or steps are in any way required for one or more particular aspects or that one or more particular aspects necessarily include logic for deciding, with or without user input or prompting, whether these features, elements and/or steps are included or are to be performed in any particular aspect.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutations of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific aspect or combination of aspects of the disclosed methods.

Disclosed in the present application is a seizure detection device and associated methods, systems, devices, and various apparatus. Example aspects of the seizure detection device can comprise a collector, a separator, and an identifier. It would be understood by one of skill in the art that the disclosed seizure detection device is described in but a few exemplary aspects among many.

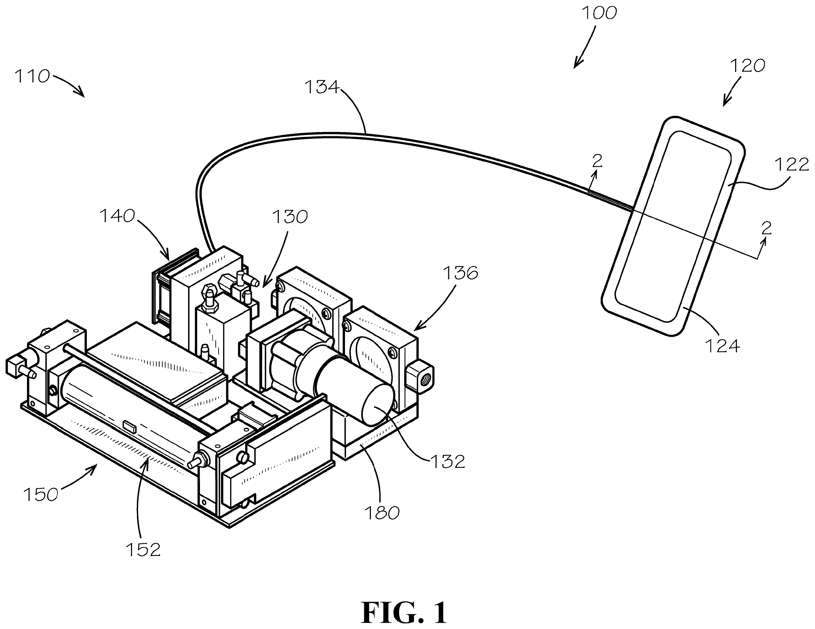

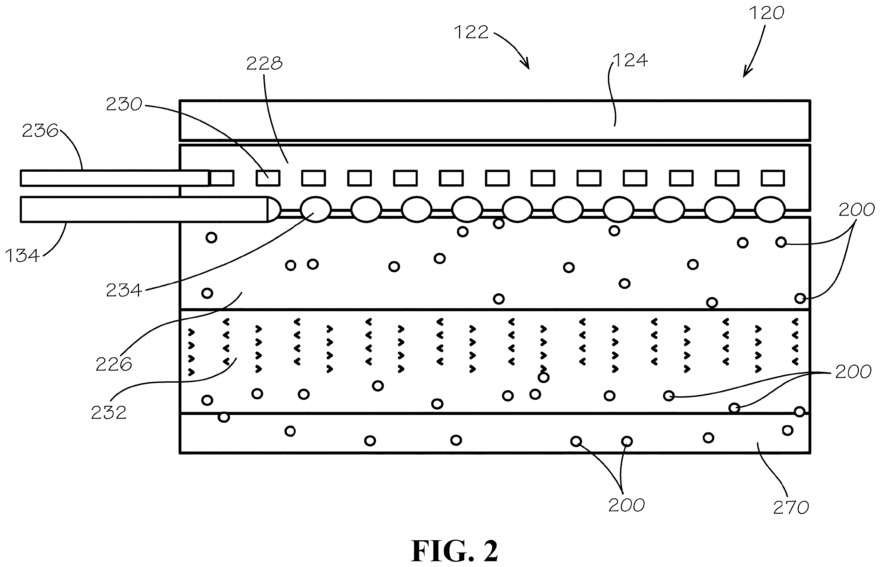

Figure 1 illustrates a first aspect of a seizure detection device 100 according to the present disclosure. The seizure detection device 100 can be configured to detect specific seizure-indicative volatile organic compounds (a.k.a. VOCs, and also known as bio-volatile compounds) than can be associated with epileptic seizure onset or occurrence in human patients. For example, the seizure-indicative VOCs can be menthone, menthyl acetate, and/or 3-ethoxy-3,7-dimethyl-1,6-octadiene, which have been identified as seizure biomarkers. In other aspects, the seizure-indicative VOCs can be any other suitable compound that can be associated with seizure in human patients. In some instances, these specific seizure-indicative VOCs may be present, either individually or in any combination, before, during, or after a seizure. Volatile organic compounds (VOCs) 200 (shown in Figure 2), including the seizure-indicative VOCs, can be emitted as gases from the human patient, for example, through the patient's skin. According to example aspects, the seizure detection device 100 can comprise a sensor device 110 that can detect and analyze VOCs 200 in a three-stage process including pre-concentration (PC), gas chromatography (GC) separation, and detection.

According to example aspects, the sensor device 110 can comprise a collector 120, a separator 130, and an identifier 150. The collector 120 can be formed as a patch 122 that can contact the patient's skin 270 (shown in Figure 2). In one aspect, the patch 122 can be adhered to the skin 270 by an adhesive. In another aspect, the patch 122 can be applied by another fastener, such as a band or tie, or any other suitable fastener known in the art. In the pre-concentration state, the collector 120 can collect target chemicals (e.g., VOCs 200) from the environment and can reject interferents. In accordance with the present invention, the collector 120 comprises a chemically-clean wrapping 124 that can isolate a collector material 226 (shown in Figure 2) from possible external environmental contaminants. The collector material 226 can be configured to collect VOCs 200 given off as a gas from the patient's skin 270, in some aspects, and may also collect other compounds. The collector material 226 can also be isolated from direct physical contact with the patient's skin 270 to minimize contamination by sweat or skin bacteria, as described in further detail below with respect to Figure 2. In accordance with the present invention, a heater 228 (shown in Figure 2) is integrated with the collector material 226 and a thermal pulse from the heater 228 can desorb the VOCs 200 (and possibly other compounds) from the collector material 226. A pump 132 of the seizure detection device 100 can then pump the desorbed VOCs 200 through a transfer tube 134 to the separator 130.

In the GC separation stage, the collected VOCs 200 can be injected into a carrier gas (not shown), such as, for example, helium or nitrogen. A small gas plug (e.g., a sample of the carrier gas and VOC mixture) can be injected into a long, rectangular flow column (not shown) of the separator 130. According to example aspects, a valve 140 can control injection of the gas plug and the direction and flow of the gas plug through the column. Example aspects of the column can be a µGC (micro gas chromatography) column, while in other aspects, the column can be a conventional GC (gas chromatography) column. In some aspects, the column can be similar to any of the aspects disclosed in U.S. Patent No. 10,151,732, filed January 11, 2016, U.S. Patent No. 6,699,392, filed June 10, 2002, and U.S. Patent No. 6,666,907, filed January 31, 2002. In some aspects, the gas plug can undergo a µGCxGC separation or a conventional GCxGC separation, which can allow for high-fidelity separations and ultra-low false alarm rates. µGCxGC separation is micro gas chromatography x micro gas chromatography separation, while GCxGC separation is a conventional gas chromatography x gas chromatography separation, both of which can also be known as two-dimensional gas chromatography.

According to example aspects, the column can be coated with a chemically-selective film, and the chemically-selective film can be referred to as a stationary phase. As the gas plug flows through the column, individual chemicals from the gas plug (including individual chemicals of the VOCs 200) diffuse into and out of the stationary phase based on their solubility within the stationary phase. VOCs 200 with a low solubility can quickly flow through the channel, while VOCs 200 with high solubility can spend a relatively long time within the stationary phase. This time-delay separates the complex chemical mixture of the gas plug into its constituent chemicals and introduces valuable spatial and chemical information that is critical for positive chemical identification and false alarm reductions in the detection stage. In example aspects, the column can be a silicon µGC column that can be about 160 cm in length, about 65 µm in width, and about 650 µm deep. In example aspects, heaters (e.g., metal heaters) (not shown) can be integrated with the silicon µGC column. Furthermore, the high aspect ratio silicon µGC column can fit on a die 136 that can be about 2 cm by 2 cm on each side of the die 136, which can be a significant size reduction in comparison to traditional columns. According to example aspects, the reduced size can allow for a µGC separation to be performed in under 30 seconds by heating the column from 70 - 200+ °C at an average power of 4.5 W.

Finally, in the detection stage, the identifier 150 can sense the chemicals eluting from the column and can transduce the chemical information to a recordable signal. For example, the identifier 150 can comprise an Ion Mobility Spectrometer (IMS) detector 152. In a particular aspect, the IMS detector 152 can be a CIMS (Correlation Ion Mobility Spectrometer) detector. In another particular aspect, the IMS detector 152 can be a LTCC (Low Temperature Co-fired Ceramic) CIMS detector. In other aspects, the detector 150 can comprise a flame ionization detector (FID), a photoionization detector (PID), a pulsed discharge ionization detectors (PDID), a resonator-based detector including quartz crystal micro balances, surface acoustic wave detectors, and/or micro-fabricated cantilever based resonators, a chemiresistor, a chemicapacitor, a thermal conductivity detector (TCD), a spectroscopic detector including vacuum ultra violet (VUV), ultraviolet, visible, and/or infrared radiation detection, a mass spectrometer detection method (MS), a non-gas chromatographic separation method such as IMS (ion mobility spectrometry), IMS-MS (ion mobility spectrometry-mass spectrometry), and/or MS-MS (tandem mass spectrometry), or any other suitable detector known in the art. Within the IMS detector 152, the incoming chemicals can be ionized and pulled down an IMS drift tube (not shown) by a potential gradient. In some aspects, the IMS drift tube can be similar to the drift tube disclosed in U.S. Patent No. 7,155,812, filed September 4, 2003.

Because the IMS detector 152 can operate at atmospheric pressures, the ionization of the chemicals can be considered a "soft" ionization, in that it minimizes the breakup or fragmentation of the chemicals. The ionized chemicals (also known as ions) can be drawn into the IMS drift tube, and the IMS drift tube can contain a faraday cup detector (not shown) at an end thereof that can count the ionic charge. The speed at which an ion travels down the IMS drift tube is a function of the ion's size, charge, and the interactions between the ion and other molecules in the IMS drift tube. Careful measurement of a characteristic transit speed down the IMS drift tube, called a reduced mobility value (or K₀), of a parent ion and its adducts can positively identify the target species (e.g., the specific seizure-indicative VOCs associated with seizures). In example aspects, the seizure detection device 100 can comprise a processor (not shown), for example, on a printed circuit board (PCB), for processing the data and determining whether one or more of the seizure-indicative VOCs is present. According to example aspects, a battery 180, such as a lithium ion battery, or another power source can be provided for powering the sensor device 110, including the processor.

When detection of one or more of the seizure-indicative VOCs is made, or detection of a significant concentration of one or more of the seizure-indicative VOCs is made, the processor can activate a signal. In some aspects, the signal can sound an immediate alarm to alert a patient that a seizure may be imminent. In some aspects, the signal can also or alternatively be sent wirelessly (e.g., via Bluetooth) to an external receiving unit, such as an application (also known as an app) on a cellular phone, smartphone, tablet, or other electronic instrument, to activate an additional alarm. In some instances, there can be enough forewarning to introduce an abortive therapy for the oncoming seizure. Furthermore, in some aspects, the seizure detection device 100 can also alert a caregiver or emergency personnel. Memory can be included in the application and/or the device 100 itself that can profile levels of seizure-indicative VOC concentration, duration, and the time and date of occurrence. This data can then be used as a diary of seizure activity for later review by the patient or a physician. In some aspects, the data can also be used to better predict future seizures based on the patient's individual chemistry pre-seizure. For example, in one aspect, the seizure detection device 100 may detect a slightly elevated concentration of menthone in the patient before multiple seizure occurrences. The processor can analyze this data to detect the pattern of increased menthone pre-seizure, and can identify increased menthone as a seizure-indicative VOC in the patient. The seizure detection device 100 can then alert the patient any time menthone, or a significant concentration of menthone, is detected.

Figure 2 illustrates a cross-sectional view of the collector 120 taken along line 2-2 of Figure 1. As shown, the collector 120 can be applied to the skin 270 of a patient. The chemically-clean wrapping 124 can define an outer layer of the collector 120. In some aspects, the chemically-clean wrapping 124 can be a polyimide film, and in the present aspect, the wrapping 124 can be a polyimide film with a silicone adhesive. In other aspects, any other suitable adhesive or other fastener can be used. The collector material 226 can define an intermediate layer of the collector 120, and in the present aspect, the collector material 226 can be formed from PDMS (polydimethylsiloxane), which is a type of silicone, for example and without limitation. In the present aspect, the heater 228 can be attached to the wrapping 124, and, in accordance with the present invention, is positioned between the wrapping 124 and the collector material 226, as shown. Alternatively and in accordance with the present invention, the heating element of the heater 228 is integrated with the wrapping 124. Furthermore, a mesh 232 can define an inner layer of the collector 120 and can be positioned between the collector material 226 and the patient's skin. Example aspects of the mesh can be formed from a polymer, such as polytetrafluoroethylene (PTFE). In other aspects, the mesh can be formed from a metal material or any other suitable material known in the art. The mesh 232 can prevent the collector material 226 from contacting the patient's skin and being contaminated by sweat, oils, and bacteria from the skin, and/or other undesirable elements.

In other aspects, the collector 120 can be configured to collect VOCs 200 through a patient's sweat, saliva, breath (e.g., exhalation), or any other suitable bodily process. Also in other aspects, the seizure-indicative VOCs can further or alternatively include β-bourbonene, β-cubebene, or any other suitable VOC that may be identified as a seizure biomarker. Furthermore, in some aspects, instead of being in contact with the patient's skin, the collector can be positioned near the patient (e.g., next to a patient's chair or bed, or elsewhere in a patient's room) and can be configured to collect VOCs from the ambient air surrounding the patient, which have been released into the air through the patient's skin and/or through the patient's exhalation.

Heater 228 comprises a heating coil 230 configured to emit a thermal pulse, which can desorb VOCs 200 received in the collector material 226 into a flow channel(s) 234 between the heating coil 230 and the collector material 226. In example aspects, a power cord 236 can be connected to the heater 228 to provide power to the heating coil 230. In some aspects, the power cord 236 can be connected to the battery 180 or other power source to transfer power to the heating coil 230. When the VOCs 200 are desorbed from the collector material 226 and into the flow channel 234, the pump 132 can then sweep the VOCs 200 out of the flow channel 234 and through the transfer tube 134 to the separator 130 (shown in Figure 1).

The seizure detection device 100 can allow patients to position themselves such that they can avoid accident, injury, embarrassment, and unnecessary trips to the emergency room. In some aspects, the seizure detection device 100 can also alert families, friends, and medical personnel to oncoming seizures, potentially reducing the amount of prophylactic medications need by patients on a daily basis. As many of these medications can be toxic and accompanied by unpleasant, occasionally life-threatening side effects, any reduction in daily dosage can result in vast improvements in patient wellbeing and functionality. Furthermore, the predictive seizure detection device 100 can allow for the development of rescue protocols in some aspects, which could reduce the severity of an oncoming seizure or, in some instances, prevent onset altogether, thus reducing or avoiding the damage that seizures can cause to the brain and the body of the patient.

Evidence indicates the presence of these seizure-indicative VOCs during the preictal (i.e., pre-seizure) stage, building in different patients at different times and at different levels of concentration based on the individual patient's metabolism and blood chemistry. Consequently, the timing of a predictive alert issued by the seizure-protection device 100 can necessarily vary from patient to patient. As a form of reference, the seizure-indicative VOCs can remain in the patient's system anywhere from about five to forty minutes postictal (i.e., post-seizure) based on the individual's metabolism.

Example aspects of the seizure detection device 100 can be on a small enough scale that the seizure detection device 100 can be easily transported with a patient as they go about daily activities, including working, exercising, eating, and sleeping. As such, various elements of the seizure detection device 100 (e.g., the column, the processor, etc.) can be formed as miniature or micro versions of such elements.

In one exemplary aspect, a collector for a seizure detection device can comprise a collector material that can be configured to collect volatile organic compounds given off from a patient's skin, a wrapping that can be configured to isolate the collector material from an external environment, a heater that can comprise a heating element, wherein the heating element can be configured to emit a thermal pulse to desorb the volatile organic compounds from the collector material, and a mesh layer that can be configured to prevent the collector material from contacting the patient's skin, wherein the collector material can be received between the wrapping and the mesh layer.

In a further exemplary aspect, the volatile organic compounds can comprise at least one of menthone, menthyl acetate, and 3-ethoxy-3,7-dimethyl-1,6-octadiene. In a further exemplary aspect, the heater can be one of integrated with the wrapping and received between the wrapping and the collector material. In a further exemplary aspect, the collector material can comprise polydimethylsiloxane. In a further exemplary aspect, the wrapping can comprise an adhesive configured to adhere the collector to the patient's skin.

In another exemplary aspect, a seizure detection device can comprise a collector comprising a collector material that can be configured to collect volatile organic compounds given off from a patient's skin, a separator comprising a gas chromatography column, wherein the gas chromatography column can comprise a chemically-selective film, and wherein mixtures of the volatile organic compounds can be configured elute from the collector and to diffuse into and out of the chemically-selective film to separate the mixtures into their constituent chemicals, and an identifier comprising a detector and a processor, wherein the detector can be configured to receive, ionize, and detect the constituent chemicals eluting from the gas chromatography column to create ionized chemicals, and wherein the processor can be configured and to process information about the ionized chemicals to identify specific volatile organic compounds indicative of a seizure.

In a further exemplary aspect, the collector can define a patch configured to contact the patient's skin, and the patch can comprise an adhesive for adhering the collector to the patient's skin. In a further exemplary aspect, the collector can comprise a chemically-clean wrapping that can be configured to isolate the collector material from external environmental contaminants. In a further exemplary aspect, the collector can further comprise a mesh layer that can be configured to isolate the collector material from the patient's skin. In a further exemplary aspect, the seizure detection device can further comprise a heater, the heater can comprise a heating element, and the heating element can be configured to emit a thermal pulse to desorb the volatile organic compounds from the collector material. In a further exemplary aspect, the seizure detection device can further comprise a pump and a transfer tube, and the pump can be configured to pump the volatile organic compounds through the transfer tube to the separator. In a further exemplary aspect, the seizure detection device can further comprise a valve that can be configured to inject a gas plug into the gas chromatography column, wherein the gas plug can comprise a carrier gas and the volatile organic compounds. In a further exemplary aspect, the detector can be an ion mobility spectrometer detector comprising a drift tube, the ionized chemicals can be configured to travel through the drift tube, and the processor can be configured to calculate a reduced mobility value of the ionized chemicals traveling through the drift tube. In a further exemplary aspect, the specific volatile organic compounds can comprise at least one of menthone, menthyl acetate, and 3-ethoxy-3,7-dimethyl-1,6-octadiene.

In another exemplary aspect, a method of detecting a seizure can comprise collecting volatile organic compounds with a collector material of a collector, separating a mixture of the volatile organic compounds into its constituent chemicals with a gas chromatography column, ionizing the constituent chemicals to create ionized chemicals and detecting the ionized chemicals, and analyzing the ionized chemicals to identify seizure-indicative volatile organic compounds.

In a further exemplary aspect, the method can further comprise transferring the volatile organic compounds from the collector to the gas chromatography column, wherein transferring the volatile organic compounds from the collector to the gas chromatography column can comprise emitting a thermal pulse from a heater to desorb the volatile organic compounds from the collector and pumping the volatile organic compounds through a transfer tube. In a further exemplary aspect, separating a mixture of the volatile organic compounds into its constituent chemicals with a gas chromatography column can comprise diffusing the volatile organic compounds into and out of a chemically-selective film of the gas chromatography column. In a further exemplary aspect, the method can further comprise injecting the volatile organic compounds into a carrier gas to form a gas plug and injecting the gas plug into the gas chromatography column. In a further exemplary aspect, analyzing the ionized chemicals to identify seizure-indicative volatile organic compounds can comprise calculating a reduced mobility value of the ionized chemicals with a processor. In a further exemplary aspect, the method can further comprise generating a signal related to the seizure-indicative volatile organic compounds with a processor.

In another exemplary aspect, a seizure detection device can comprise a collector that can be configured to collect volatile organic compounds given off from a patient's skin, a separator that can be configured to separate mixtures of the volatile organic compounds into their constituent chemicals, and an identifier that can be configured to ionize the constituent chemicals to create ionized chemicals and to process information about the ionized chemicals to identify specific volatile organic compounds indicative of a seizure.

One should note that conditional language, such as, among others, "can," "could," "might," or "may," unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more particular embodiments or that one or more particular embodiments necessarily include logic for deciding, with or without user input or prompting, whether these features, elements and/or steps are included or are to be performed in any particular embodiment.

It should be emphasized that the above-described embodiments are merely possible examples of implementations, merely set forth for a clear understanding of the principles of the present disclosure. Any process descriptions or blocks in flow diagrams should be understood as representing modules, segments, or portions of code which include one or more executable instructions for implementing specific logical functions or steps in the process, and alternate implementations are included in which functions may not be included or executed at all, may be executed out of order from that shown or discussed, including substantially concurrently or in reverse order, depending on the functionality involved, as would be understood by those reasonably skilled in the art of the present disclosure.

In an example, there is disclosed a collector for a seizure detection device comprising: a collector material configured to collect volatile organic compounds given off from a patient's skin; a wrapping configured to isolate the collector material from an external environment; a heater comprising a heating element, the heating element configured to emit a thermal pulse to desorb the volatile organic compounds from the collector material; and a mesh layer configured to prevent the collector material from contacting the patient's skin, wherein the collector material is received between the wrapping and the mesh layer.

In an example, the volatile organic compounds comprise at least one of menthone, menthyl acetate, and 3-ethoxy-3,7-dimethyl-1,6-octadiene.

In an example, the heater is one of integrated with the wrapping and received between the wrapping and the collector material.

In an example, the collector material comprises polydimethylsiloxane.

In an example, the wrapping comprises an adhesive configured to adhere the collector to the patient's skin.

In an example, there is disclosed a seizure detection device comprising: a collector comprising a collector material configured to collect volatile organic compounds given off from a patient's skin; a separator comprising a gas chromatography column, the gas chromatography column comprising a chemically-selective film, wherein mixtures of the volatile organic compounds are configured elute from the collector and to diffuse into and out of the chemically-selective film to separate the mixtures into their constituent chemicals; and an identifier comprising a detector and a processor, the detector configured to receive, ionize, and detect the constituent chemicals eluting from the gas chromatography column to create ionized chemicals, the processor configured and to process information about the ionized chemicals to identify specific volatile organic compounds indicative of a seizure.

In an example, the collector defines a patch configured to contact the patient's skin, the patch comprising an adhesive for adhering the collector to the patient's skin.

In an example, the collector comprises a chemically-clean wrapping configured to isolate the collector material from external environmental contaminants.

In an example, the collector further comprises a mesh layer configured to isolate the collector material from the patient's skin.

In an example, the seizure detection device further comprises a heater comprising a heating element, the heating element configured to emit a thermal pulse to desorb the volatile organic compounds from the collector material.

In an example, the seizure detection device further comprises a pump and a transfer tube, the pump configured to pump the volatile organic compounds through the transfer tube to the separator.

In an example, the seizure detection device further comprises a valve configured to inject a gas plug into the gas chromatography column, wherein the gas plug comprises a carrier gas and the volatile organic compounds.

In an example, the detector is an ion mobility spectrometer detector comprising a drift tube; the ionized chemicals are configured to travel through the drift tube; and the processor is configured to calculate a reduced mobility value of the ionized chemicals traveling through the drift tube.

In an example, the specific volatile organic compounds comprise at least one of menthone, menthyl acetate, and 3-ethoxy-3,7-dimethyl-1,6-octadiene.

In an example not forming part of the claimed invention , there is disclosed a method of detecting a seizure comprising:
collecting volatile organic compounds with a collector material of a collector; separating a mixture of the volatile organic compounds into its constituent chemicals with a gas chromatography column; ionizing the constituent chemicals to create ionized chemicals and detecting the ionized chemicals; and analyzing the ionized chemicals to identify seizure-indicative volatile organic compounds.

In an example, the method further comprises transferring the volatile organic compounds from the collector to the gas chromatography column, wherein transferring the volatile organic compounds from the collector to the gas chromatography column comprises: emitting a thermal pulse from a heater to desorb the volatile organic compounds from the collector; and pumping the volatile organic compounds through a transfer tube.

In an example, separating a mixture of the volatile organic compounds into its constituent chemicals with a gas chromatography column comprises diffusing the volatile organic compounds into and out of a chemically-selective film of the gas chromatography column.

In an example, the method further comprises injecting the volatile organic compounds into a carrier gas to form a gas plug and injecting the gas plug into the gas chromatography column.

In an example, analyzing the ionized chemicals to identify seizure-indicative volatile organic compounds comprises calculating a reduced mobility value of the ionized chemicals with a processor.

In an example, the method further comprises generating a signal related to the seizure-indicative volatile organic compounds with a processor.

In an example, there is disclosed a seizure detection device comprising: a collector configured to collect volatile organic compounds given off from a patient's skin; a separator configured to separate mixtures of the volatile organic compounds into their constituent chemicals; and an identifier configured to ionize the constituent chemicals to create ionized chemicals and to process information about the ionized chemicals to identify specific volatile organic compounds indicative of a seizure.

## Claims

1. A seizure detection device (100) comprising:
a collector (120) comprising a collector material (226) configured to collect volatile organic compounds given off from a patient's skin wherein the collector (120) comprises a chemically-clean wrapping (124) configured to isolate the collector material from external environmental contaminants;
a separator (130) comprising a gas chromatography column, the gas chromatography column comprising a chemically-selective film, wherein mixtures of the volatile organic compounds are configured to elute from the collector and to diffuse into and out of the chemically-selective film to separate the mixtures into their constituent chemicals;
an identifier (150) comprising a detector (152) and a processor, the detector configured to receive, ionize, and detect the constituent chemicals eluting from the gas chromatography column to create ionized chemicals, the processor configured to process information about the ionized chemicals to identify specific volatile organic compounds indicative of a seizure; and
a heater (228) comprising a heating coil (230) and one or more flow channels (234) between the heating coil and the collector material, the heating coil configured to emit a thermal pulse to desorb the volatile organic compounds received in the collector material into the one or more flow channels , wherein the heater is either integrated with the wrapping or received between the wrapping and the collector material.

2. The seizure detection device of claim 1, wherein the collector defines a patch (122) configured to contact the patient's skin, the patch comprising an adhesive for adhering the collector to the patient's skin.

3. The seizure detection device of claim 1, wherein the collector further comprises a mesh layer (232) configured to isolate the collector material from the patient's skin.

4. The seizure detection device of claim 1, further comprising a pump (132) and a transfer tube (134), the pump configured to pump the volatile organic compounds through the transfer tube to the separator.

5. The seizure detection device of claim 1, further comprising a valve (140) configured to inject a gas plug into the gas chromatography column, wherein the gas plug comprises a carrier gas and the volatile organic compounds.

6. The seizure detection device of claim 1, wherein:
the detector is an ion mobility spectrometer detector comprising a drift tube;
the ionized chemicals are configured to travel through the drift tube; and
the processor is configured to calculate a reduced mobility value of the ionized chemicals traveling through the drift tube.

7. The seizure detection device of claim 1, wherein the specific volatile organic compounds comprise at least one of menthone, menthyl acetate, and 3-ethoxy-3,7-dimethyl-1,6-octadiene.

8. The seizure detection device of claim 1, wherein the collector material comprises polydimethylsiloxane.

9. The seizure detection device of claim 1, wherein the wrapping comprises an adhesive configured to adhere the collector to the patient's skin.

## Patentansprüche

1. Vorrichtung zur Erkennung von Anfällen (100), umfassend:
Einen Kollektor (120), der ein Kollektormaterial (226) umfasst, das konfiguriert ist, um flüchtige organische Verbindungen aufzufangen, die von der Haut eines Patienten abgegeben werden, wobei das Kollektorelement (120) eine chemisch reine Umhüllung (124) umfasst, die konfiguriert ist, um das Kollektormaterial vor Verunreinigungen aus der äußeren Umgebung zu schützen;
einen Separator (130), der eine Gaschromatographiesäule umfasst, wobei die Gaschromatographiesäule einen chemisch selektiven Film umfasst, wobei Mischungen der flüchtigen organischen Verbindungen konfiguriert sind, um aus dem Kollektor zu eluieren und in den chemisch selektiven Film hinein- und aus diesem herauszudiffundieren, um die Mischungen in ihre einzelnen chemischen Bestandteile zu trennen;
einen Identifikator (150), der einen Detektor (152) und einen Prozessor umfasst, wobei der Detektor konfiguriert ist, um die aus der Gaschromatographiesäule eluierenden chemischen Bestandteile aufzunehmen, zu ionisieren und zu erfassen, um ionisierte chemische Stoffe zu erzeugen, und der Prozessor konfiguriert ist, um Informationen über die ionisierten chemischen Stoffe zu verarbeiten, um bestimmte flüchtige organische Verbindungen zu identifizieren, die auf einen Anfall hindeuten; und
einen Heizer (228), der eine Heizspule (230) und einen oder mehrere Strömungskanäle (234) zwischen der Heizspule und dem Kollektormaterial umfasst, wobei die Heizspule konfiguriert ist, um einen Wärmeimpuls abzugeben, um die im Kollektormaterial aufgenommenen flüchtigen organischen Verbindungen in den einen oder die mehreren Strömungskanäle zu desorbieren, wobei der heizer entweder in die Umhüllung integriert ist oder zwischen der Umhüllung und dem Kollektormaterial angeordnet ist.

2. Vorrichtung zur Erkennung von Anfällen nach Anspruch 1, wobei der Kollektor ein Pflaster (122) definiert, das konfiguriert ist, um mit der Haut des Patienten in Kontakt zu kommen, wobei das Pflaster einen Klebstoff zum Kleben des Kollektors auf der Haut des Patienten umfasst.

3. Vorrichtung zur Erkennung von Anfällen nach Anspruch 1, wobei der Kollektor ferner eine Netzschicht (232) umfasst, die konfiguriert ist, um das Kollektormaterial von der Haut des Patienten zu isolieren.

4. Vorrichtung zur Erkennung von Anfällen nach Anspruch 1, die ferner eine Pumpe (132) und einen Förderschlauch (134) umfasst, wobei die Pumpe konfiguriert ist, um die flüchtigen organischen Verbindungen durch den Förderschlauch zum Separator zu pumpen.

5. Vorrichtung zur Erkennung von Anfällen nach Anspruch 1, die ferner ein Ventil (140) umfasst, das konfiguriert ist, um einen Gaspfropfen in die Gaschromatographiesäule einzuspritzen, wobei der Gaspfropfen ein Trägergas und die flüchtigen organischen Verbindungen umfasst.

6. Vorrichtung zur Erkennung von Anfällen nach Anspruch 1, wobei:
der Detektor ein Ionenmobilitätsspektrometerdetektor ist, der ein Driftröhrchen umfasst;
die ionisierten Chemikalien konfiguriert sind, um sich durch das Driftröhrchen zu bewegen; und
der Prozessor konfiguriert ist, um einen Wert für die reduzierte Mobilität der sich durch das Driftröhrchen bewegenden ionisierten Chemikalien zu berechnen.

7. Vorrichtung zur Erkennung von Anfällen nach Anspruch 1, wobei die spezifischen flüchtigen organischen Verbindungen mindestens eines von Menthon, Menthylacetat und 3-Ethoxy-3,7-dimethyl-1,6-octadien umfassen.

8. Vorrichtung zur Erkennung von Anfällen nach Anspruch 1, wobei das Kollektormaterial Polydimethylsiloxan umfasst.

9. Vorrichtung zur Erkennung von Anfällen nach Anspruch 1, wobei die Umhüllung einen Klebstoff umfasst, der konfiguriert ist, um den Kollektor auf der Haut des Patienten zu befestigen.

## Revendications

1. Dispositif de détection des crises (100) comprenant :
un collecteur (120) comprenant un matériau collecteur (226) conçu pour recueillir des composés organiques volatils dégagés par la peau d'un patient, dans lequel le collecteur (120) comprend un emballage chimiquement propre (124) conçu pour isoler le matériau collecteur des contaminants environnementaux externes ;
un séparateur (130) comprenant une colonne de chromatographie en phase gazeuse, la colonne de chromatographie en phase gazeuse comprenant un film chimiquement sélectif, dans lequel les mélanges de composés organiques volatils sont conçus pour s'éluer du collecteur et se diffuser à l'intérieur et à l'extérieur du film chimiquement sélectif afin de séparer les mélanges en leurs composants chimiques constitutifs ;
un identifiant (150) comprenant un détecteur (152) et un processeur, le détecteur étant configuré pour recevoir, ioniser et détecter les composants chimiques constitutifs éluant de la colonne de chromatographie en phase gazeuse afin de créer des substances chimiques ionisées, le processeur étant configuré pour traiter les informations relatives aux substances chimiques ionisées afin d'identifier des composés organiques volatils spécifiques indiquant une crise ; et
un dispositif de chauffage (228) comprenant un serpentin chauffant (230) et un ou plusieurs canaux d'écoulement (234) entre le serpentin chauffant et le matériau collecteur, le serpentin chauffant étant configuré pour émettre une impulsion thermique afin de désorber les composés organiques volatils reçus dans le matériau collecteur vers le ou les canaux d'écoulement, dans lequel le dispositif de chauffage est soit intégré à l'enveloppe, soit reçu entre l'enveloppe et le matériau collecteur.

2. Dispositif de détection de crises selon la revendication 1, dans lequel le collecteur définit un patch (122) conçu pour entrer en contact avec la peau du patient, le patch comprenant un adhésif permettant de fixer le collecteur sur la peau du patient.

3. Dispositif de détection de crises selon la revendication 1, dans lequel le collecteur comprend en outre une couche maillée (232) conçue pour isoler le matériau collecteur de la peau du patient.

4. Dispositif de détection de crises selon la revendication 1, comprenant en outre une pompe (132) et un tube de transfert (134), la pompe étant configurée pour pomper les composés organiques volatils à travers le tube de transfert vers le séparateur.

5. Dispositif de détection de crises selon la revendication 1, comprenant en outre une vanne (140) configurée pour injecter un bouchon de gaz dans la colonne de chromatographie en phase gazeuse, dans lequel le bouchon de gaz comprend un gaz vecteur et les composés organiques volatils.

6. Dispositif de détection de crises selon la revendication 1, dans lequel :
le détecteur est un détecteur de type spectromètre à mobilité ionique comprenant un tube à dérive ;
les substances chimiques ionisées sont conçues pour se déplacer à travers le tube à dérive ; et
le processeur est configuré pour calculer une valeur de mobilité réduite des substances chimiques ionisées se déplaçant à travers le tube de dérive.

7. Dispositif de détection de crises selon la revendication 1, dans lequel les composés organiques volatils spécifiques comprennent au moins l'un parmi la menthone, l'acétate de menthyle et le 3-éthoxy-3,7-diméthyl-1,6-octadiène.

8. Dispositif de détection de crises selon la revendication 1, dans lequel le matériau collecteur comprend du polydiméthylsiloxane.

9. Dispositif de détection de crises selon la revendication 1, dans lequel l'enveloppe comprend un adhésif conçu pour fixer le collecteur sur la peau du patient.
